# EUROPEAN PATENT SPECIFICATION

(11) **EP 0 748 335 B1**
(45) Date of publication and mention of the grant of the patent: **14.08.2002**
(21) Application number: 95910002.5
(22) Date of filing: 28.02.1995
(51) Int. Cl.: C07K 14/04, C12N 1/20, C12P 21/00

(54) **ANTIBIOTIC STALOBACINS**
ANTIBIOTISCH WIRKSAME STALOBACINE
STALOBACINES A CARACTERE ANTIBIOTIQUE

(30) Priority: 01.03.1994 JP 3121994
(43) Date of publication of application: 18.12.1996
(73) Proprietor: SHIONOGI & CO., LTD., Osaka-shi, Osaka 541-0045 (JP)
(72) Inventor: YAGI, Shigeo, Osaka 569 (JP); KAGEYAMA, Bunji, Osaka 567 (JP); KAWAMURA, Yoshimi, Osaka 562 (JP); MATSUMOTO, Koichi, Osaka 560 (JP); KAMIGAUCHI, Toshiyuki, Osaka 565 (JP); MATSUTANI, Shigeru, Wakayama 648 (JP); KAMATA, Susumu, Hyogo 665 (JP)
(74) Representative: Baverstock, Michael George Douglas
(86) International application number: JP9500314
(87) International publication number: WO9523812

(56) References cited:
- CA-A- 2 104 847
- DATABASE WPI Section Ch, Week 9105 Derwent Publications Ltd., London, GB; Class B04, AN 91-033526 & JP,A,02 303 496 (SHIONOGI KK) , 17 December 1990

## Description

The present invention relates to novel antibiotics. In particular, this invention relates to antibiotic stalobacins H and I produced by *Pseudomonas* sp. PBJ-5360-STR-1-21, the microorganism which produces the antibiotics, and a process for producing the same.

It is well known that antibacterial activity of a particular antibiotic varies depending on a nature of bacteria to be treated and the effect of the antibiotic often reduces because of the advent of resistant strains. The advent of multiple drug resistant bacteria has recently become a big problem. Accordingly, development of novel and effective antibiotics has been desired for performing effective treatments. Above all, many Gram-positive bacteria, such as *Staphylococcus*, hemolytic *streptococcus* or the like are resistant to antibiotics, and there is continuous need for the development of novel antibiotics having high potency to these Gram-positive bacteria.

The present invention provides antibiotic stalobacins selected from the group consisting of antibiotic stalobacins H and I produced by the above-noted *Pseudomonus* sp. PBJ-5360-STR-1-21. These antibiotics are peptide antibiotics which are produced by said *Pseudomonas* sp. PBJ-5360-STR-1-21. Stalobacins H and I (hereinafter sometimes referred to as merely "stalobacins") are obtained in the form of a mixture of closely related analogs by cultivating said *Pseudomonas.* Their antibacterial activities are much more potent than those of known antibiotics. Stalobacins H and I have physico-chemical properties as shown in the following Table 1.

**Table 1**

| Physico-chemical Properties of Stalobacins H and I: | | |
|---|---|---|
| | Stalobacin H | Stalobacin I |
| m.p. (°C) (as Na salt) | 235°C (dec.) | 240°C (dec.) |
| LSI-MS Protonated | 1396 | 1325 |
| molecular ion (m/z) | | |
| IR (KBr) (cm⁻¹) | 3374, 1747, | 3387, 1747, |
| | 1654, 1597, 1525 | 1651, 1596, 1527 |
| UV (H₂O) | End absorption | End absorption |
| CD (H₂O) | [θ]₁₉₄-66980 | |
| | [θ]₂₁₂+9851 | [θ]₂₀₆+11530 |
| | [θ]₂₃₂-31520 | [θ]₂₃₂-28660 |
| | [θ]₂₅₇+4288 | [θ]₂₅₇+4749 |
| Retention time (min.) | 8.8 | 9.7 |
| in HPLC* | | |
| Amino Acid Analysis | | |
| (molar ratio) | | |
| HyAsp¹⁾ | HyAsp (1) | HyAsp (1) |
| Asp | Asp (1) | Asp (1) |
| Ser | Ser (1) | Ser (1) |
| HyIle²⁾ | HyIle (1) | HyIle (1) |
| Gly | Gly (1) | Gly (1) |
| Ala | Ala (1) | --- |

| | | |
|---|---|---|
| * Column: Develosil 5C18, 4.6 i.d. x 250 mm Mobile phase: CH₃CN/2 mM H₃PO₄ (containing 50 mM Na₂SO₄) = 43/57 Flow rate: 1 ml/min. | | |
| 1) Hydroxyaspartic acid | | |
| 2) Hydroxyisoleucine | | |

The antibiotic stalobacins of the present invention characterized by the above properties have been found to have excellent antibacterial activities *in vitro* and *in vivo*, showing potent effects especially on Gram-positive bacteria.

Thus, the antibiotic stalobacins of the present invention show excellent activities, and have higher activities especially against Gram-positive bacteria, as shown below in Table 2.

No death was observed in acute toxity test by intravenous administration of 300 mg/kg and 500 mg/kg of the antibiotic stalobacins to mice.

Antibiotic stalobacins H and I of the present invention are produced by cultivating *Pseudomonas* sp. PBJ-5360-STR-1-21, a variant derived from *Pseudomonas* sp. PBJ-5360 (BIKOKEN deposition No. 10578, FERM BP-4342), which produces a mixture of stalobacins, aerobically in a liquid medium containing assimilable carbon sources, nitrogen sources and mineral salts in a conventional manner. This bacterium has been identified as the above mentioned strain by cultivating it according to the method as hereinafter described in Experiment 2 and examining comprehensively its morphology, culture properties, physiological and biochemical properties in reference to the description of Bergey's Manual of Systematic Bacteriology, Vol. 1 (1984). This strain may undergo a spontaneous or artificial mutation, and it is obvious to a person skilled in the art that such mutants should be included in the scope of the present invention, as far as they retain an ability to produce the stalobacins of the present invention. Thus, the present invention provides also *Pseudomonas* sp. PBJ-5360-STR-1-21 producing novel antibiotic stalobacins H or I and mutants thereof having an ability to produce said antibiotic stalobacins. *Pseudomonas* sp. PBJ-5360-STR-1-21 was deposited under accession No. FERM P-14149 with National Institute of Bioscience and Human Technology, Higashi 1-1-3, Tsukubashi, Ibaraki Pref. Japan, on February 16, 1994, and said original deposit was transferred to International deposition under Budapest Treaty on April 28, 1994, and given Accession number FERM BP-4661.

Furthermore, the present invention provides a process for producing antibiotic stalobacins H and I by cultivating such strain.

Ordinary compositions of medium and ordinary conditions used for conventional cultivation for producing antibiotics can be adopted. In principle, the medium includes carbon sources, nitrogen sources, mineral salts and the like. If necessary, vitamins, precursors or the like can be added. Examples of carbon sources are glucose, starch, dextrin, glycerin, molasses, organic acids and the like, and these carbon sources may be used alone or in a mixture thereof. Examples of nitrogen sources are soybean powder, corn steep liquor, meat extract, yeast extract, cotton seed powder, peptone, wheat embryos, ammonium sulfate, ammonium nitrate and the like, and these nitrogen sources may be used alone or in a mixture thereof. Examples of mineral salts are calcium carbonate, sodium chloride, potassium chloride, magnesium sulfate, cupric sulfate, manganese chloride, zinc sulfate, cobalt chloride, various phosphates and the like. These mineral salts may be added to a medium when required. A sufficient amount of antibiotic stalobacins H and I is produced by cultivating *Pseudomonas* sp. PBJ-5360-STR-1-21 of the present invention in an appropriate medium at temperatures from 20 to 35°C, preferably 25 to 29°C, for about 1 to 7 days. The product is then isolated and purified, if necessary, from the culture in a conventional manner. All of such procedures are well known to a person skilled in the art.

The antibiotic stalobacins of the present invention are believed to be useful for treating various infections, in particular, treating infections caused by multiple drug-resistant Gram-positive bacteria, since they exhibit marked antibacterial activities *in vivo* and *in vitro*.

Figure 1 is a graph showing IR spectrum of stalobacin H.

Figure 2 is a graph showing IR spectrum of stalobacin I.

Figure 3 is a graph showing NMR spectrum of stalobacin H.

Figure 4 is a graph showing MMR spectrum of stalobacin I.

The present invention will be explained in more detail below by illustrating Examples and Experiments.

### Example 1

### (a) Fermentation Step:

Eight hundred ml of a medium (adjusted to pH 7 with 2N-NaOH) consisting of 1.0% glucose, 0.5% yeast extract (Difco) and tap water in a 2 L Erlenmeyer flask was inoculated with a seed strain of *Pseudomonas* sp. PBJ-5360-STR-1-21 (kept at -80°C in a 2 ml vial), and the resultant mixture was subjected to a shaking cultivation at 180 rpm with 70 mm of shaking breadth at 28°C for 22 hours. The culture (800 ml) was inoculated to 20 L of a medium (adjusted to pH 7 with 2N-NaOH) containing 1.0% glucose, 0.4% yeast extract (Difco), 1.0% malt extract (Difco), 0.1% polypeptone (Nippon Seiyaku) and tap water in a 30 L jar fermenter, and the resultant mixture was cultivated with agitation at 200 rpm, with 14 L/min of aeration under 0.35 kg/cm²G of inner pressure, at 28°C for 21 hours.

Then, 8 L of this culture was inoculated to 125 L of a medium (adjusted to pH 7 with 2N-NaOH) consisting of 2.0% soluble starch, 2.0% powdery yeast, 1.5% β-cyclodextrin, 0.5% olive oil, 0.3% magnesium chloride•6H₂O, 0.1% potassium dihydrogenphosphate, 0.0008% antifoaming reagent ADECANOL (Asahi Denka Kogyo) LG109 and tap water in a 250 L tank, and the resultant mixture was cultivated with 65 L/min of aeration, under 0.35 kg/cm²G of inner pressure, with agitation at 350 rpm at 28°C for 72 hours.

### (b) Separation Step:

To 138 L of the culture obtained in the foregoing step was added 1.4 L of chloroform for sterilization. Then, 15 L of Amberlite XAD-7 (Organo) was added and the resulting mixture was mixed with stirring for 3 hours for a batch adsorption of the active substances onto the resin. The resin was recovered using a #140 mesh stainless steel sieve. The resin was washed with water, put in a glass column (inner diameter: 20 cm), washed with 40 L of water, 40 L of 30% methanol and then 15 L of 50% methanol in 20 mM phosphate buffer (pH 7.5), and the active substances were eluted by 40 L of 60% methanol in 20 mM phosphate buffer (pH 7.5).

Fractions (20 L) showing antibacterial activity to S. *aureus* JC-1 were collected, concentrated *in vacuo* to 3 L. The concentrate was washed with 3 L of ethyl acetate to remove lipophilic materials. The ethyl acetate contained in the aqueous layer was evaporated *in vacuo*. The active substances were adsorpted on Amberlite XAD-7 (Organo) in a 1 L column (inner diameter 6.5 cm) and the resin was washed with 2 L of water. The elution was carried out using 2 L of 30% aqueous MeOH and 3 L of 50% aqueous MeOH. The eluted fractions were subjected to a HPLC analysis, and the fractions containing stalobacins were collected, adjusted to pH 7.0 with 2N-HCl, concentrated *in vacuo* and lyophilized to obtain 3890 mg of powder.

### (c) Purification Step:

### The First Purification Step:

One thousand and eight hundred twenty mg of the crude powder obtained in the foregoing step was dissolved in 60 ml of 20 mM phosphate buffer (pH 7.5). The solution was subjected to a preparative high-speed liquid chromatography using YMC ODS column [S-15/30 µ, 5.0 x 50 cm, eluent: acetonitrile/20 mM phosphate buffer (pH 7.5). 50 mM sodium sulfate = 40/60, flow rate: 50 ml/min, UV detection; 210 nm] to obtain a fraction (2.4 L) containing stalobacins H and I as main ingredients. The acetonitrile in the fraction was distilled off and the residue was passed through a column of Diaion HP-20 (Mitsubishi Kasei Corporation). The resin was washed with water, and the adsorbed components were eluted with 60% aqueous acetone. The acetone in the eluate was distilled away *in vacuo* and the residue was lyophilized to obtain 126 mg of powder.

### The Second Purification Step:

The powder obtained in the above step was purified by preparative high-speed liquid chromatography under the following conditions to obtain stalobacins H and I.

The powder (126 mg) was dissolved in 7 ml of 50 mM phosphate buffer (pH 7.0). Fifteen mg of the sample per one procedure was charged into Asahipak ODP-90 column (inner diameter 21.5 mm x 300 mm, eluent: acetonitrile solution of 20 mM AcOH/aqueous solution of 20 mM AcOH = 40/60, flow rate: 8 ml/min, UV detection: 220 nm), and stalobacin H was collected from the fractions of 144 ml to 184 ml and stalobacin I was collected from the fractions of 216 ml to 288 ml. This fractionation was repeated, and collected fractions were neutralized to pH 7.0 with aqueous 1N-NaOH. The acetonitrile was distilled off *in vacuo* and NaCl was added to the residue to obtain 5% NaCl concentration. The resultant mixture was adjusted to pH 7.5 with 1N NaOH and passed through MCI GEL CHP20P column (75 to 150 µ, Mitsubishi Kasei) which had been equilibrated with 5% aqueous NaCl solution. The column was washed with water and eluted with 70% aqueous MeOH. The MeOH in the eluate was distilled off *in vacuo* and the residue was lyophilized to give 12 mg of pure stalobacin H and 38 mg of pure stalobacin I.

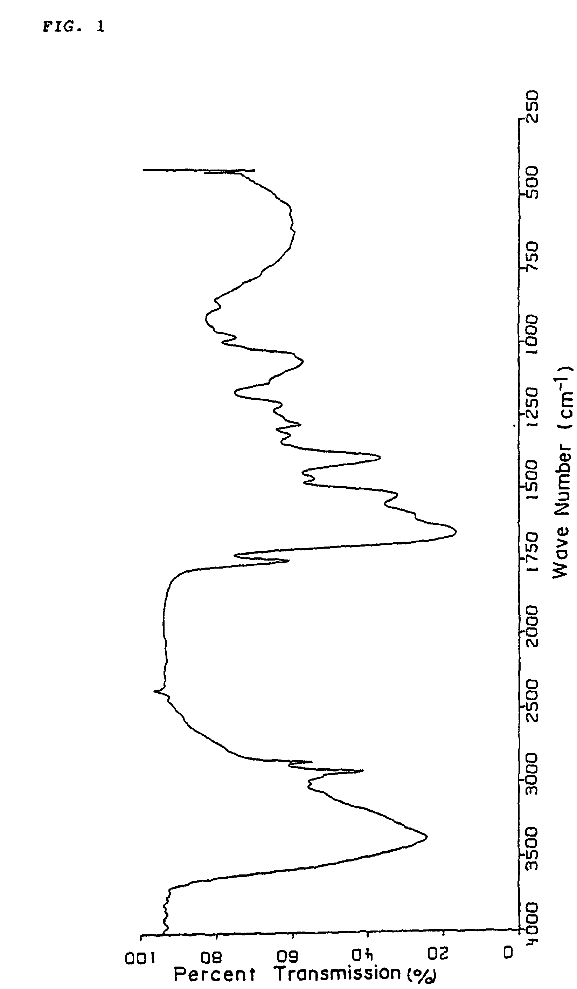

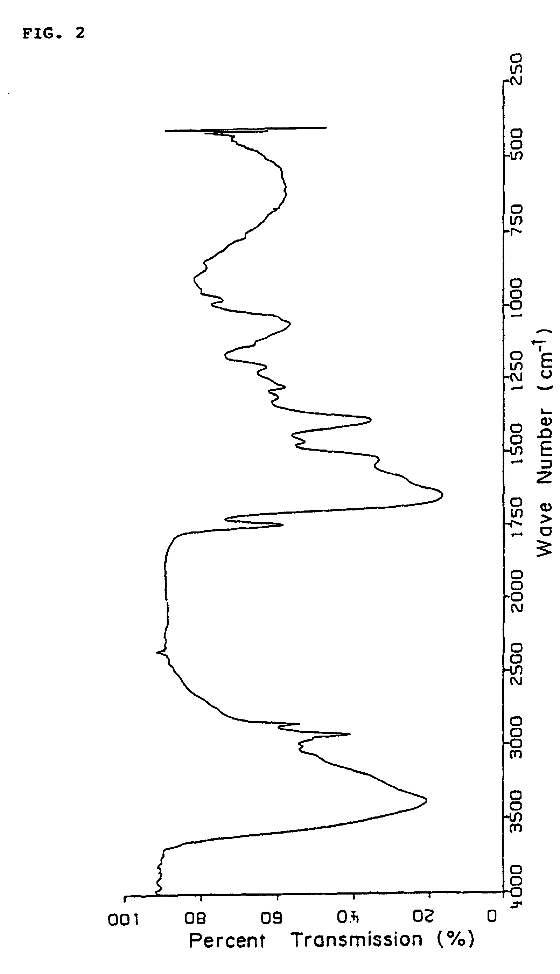

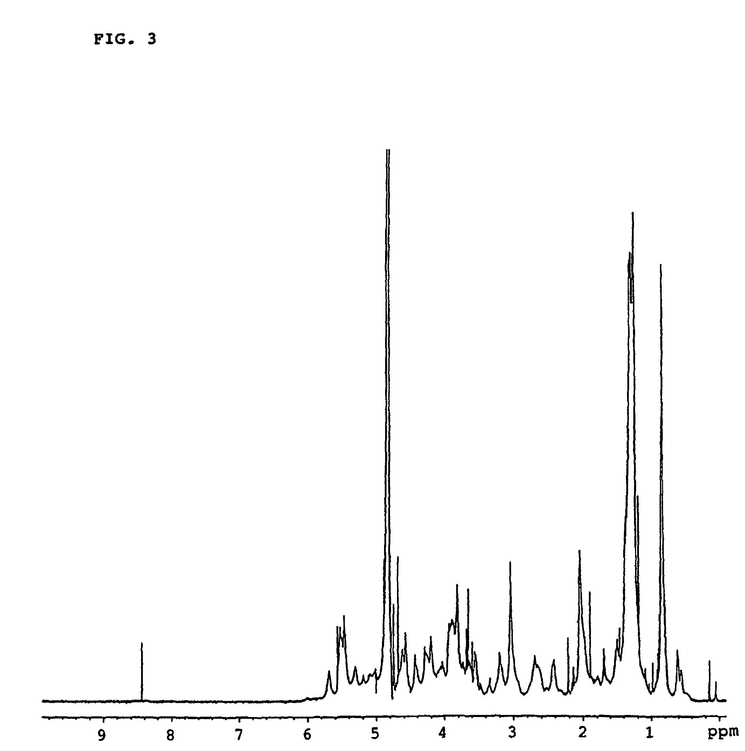

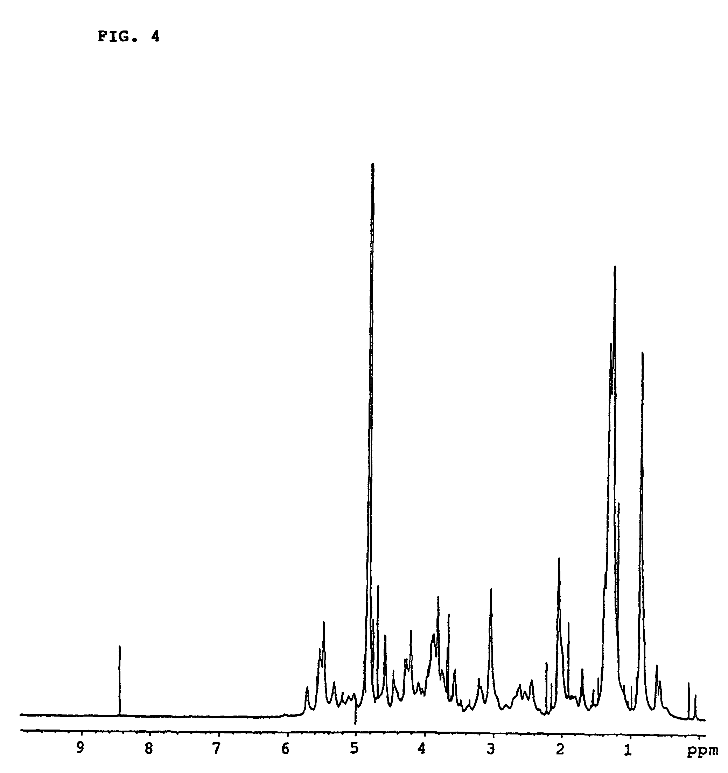

Physico-chemical properties of stalobacins H and I obtained in Example 1 are shown in Table 2. IR spectra of stalobacins H and I were shown in Figs. 1 and 2 respectively, and NMR spectra of stalobacins H and I were shown in Figs. 3 and 4 respectively.

### Experiments 1 Antibacterial Activity in vitro and in vivo

### 1) In vitro Antibacterial Activity:

Antibacterial activity *in vitro* of antibiotic stalobacins H and I obtained in Example 1 was assayed by the agar dilution method. The results are shown in Table 2.

**Table 2**

| | Stalobacin (µg/ml), 10⁶ cfu/ml) | |
|---|---|---|
| | H | I |
| Gram-positive bacteria | | |
| *S. aureus* FDA JC-1 | 0.1 | 0.05 |
| *S. faecalis* SR1004 | 0.2 | 0.2 |
| *S. aureus* 3626 (MRSA) | 0.1 | 0.1 |

### 2) In vivo Antibacterial Activity:

Antibacterial activity *in vivo* of stalobacin I was assayed. Mice were intraperitoneally challenged with infectious bacteria. One hour after the challenge, the test compound was subcutaneously administered. ED₅₀ values was calculated on the basis of survival ratio on 7th day after the challenge. MIC was determined according to the agar dilution method. The results are shown in Table 3.

**Table 3**

| Protective Effect of Stalobacin I in Mice Systemically Infected: | | |
|---|---|---|
| | ED₅₀ (mg/kg) | MIC (µg/ml) |
| | Stalobacin I | Stalobacin I |
| *S. aureus* SR3637 (H-MRSA) | 0.12 | 0.012 |
| *S. pneumoniae* Type I | 0.046 | 0.006 |
| *E. faecalis* SR1004 | 1.50 | 0.2 |

### Experiment 2 Bacteriological Properties of PBJ-5360 and PBJ-5360-STR-1-21:

PBJ-5360-STR-1-21 of the present invention was obtained as a mutant of the above-mentioned PBJ-5360 strain. PBJ-5360 was isolated from the soil collected in Kyoto, Japan. Various bacteriological properties of PBJ-5360-STR-1-21 of the present invention are shown below. Cultivation was effected at 28°C in principle.

### A. Morphology:

It is a Gram-negative rod. Its size is 0.3 - 0.5 (µm) x 0.8 - 1.3 (µm). It vigorously moves with one or more polar flagella.

### B. Characteristics of Culture

### 1) Cultivation in meat infusion medium:

Growth of the bacteria was hardly observed. Off white translucent precipitates formed very slightly at the bottom of the test tube.

### 2) Meat infusion agar stab culture:

Growth in thread form or small nipple form along the stab line was observed. Neither evolution of gas nor production of pigment was observed. Reddish thin bacterial plaque appeared on the surface, but this bacterial plaque became translucent and light brown with the lapse of time and verrucose projections were observed in several places. It is an aerobic bacterium.

### 3) Meat infusion agar slant culture:

The growth of the bacteria was not so rapid and began at 28°C after two days (observed with naked eyes). The bacteria grew in thread form, and its bacterial plaque was translucent and light yellow with flat swelling having spotty appearance. The periphery was whole peripheral. Then, the bacterial plaque grew favorably in thread form or verrucose form with gloss. Thus, a wet slightly reddished translucent brown bacterial plaque was obtained. The periphery was wavy or long wavy. Production of any gas or pigment was not observed.

### 4) Meat infusion gelatin stab culture:

Cultivation was effected at room temperature (22 - 25°C). The gelatin was slightly liquefied.

### 5) Cultivation on the meat infusion agar plane medium:

The growth of the bacteria was not so rapid. The colony became visible at 28°C after two days. The colony was initially small, spotty, translucent and brown with a whole periphery. The colony was two small to be observed about its swelling. Then, the colony grew in a spotty or circular form and with whole periphery and the swelling was flat or convex circular. The colony was translucent and brown with gloss. Neither gas nor soluble pigment was produced.

### 6) Characteristics in litmus milk culture:

An acid formation did not occur, and peptonization occurred but the reaction began after 14 days. Thus, the reaction was rather slowly. No gas evolved.

### C. Physiological and Biochemical Properties

- 1): Catalase test: positive
- 2): Oxidase test: positive
- 3): OF-test: negative (showing to be alkaline)
- 4): Hemolytic test: positive (weakly)
- 5): Viability at 5°C: negative
- 6): Production of H₂S: negative
- 7): Nitrate reduction: positive
- 8): Denitrification: negative (although no nitrogen gas was evolved, it seemed to reduce No₂⁻)
- 9): Citrate utilization: negative (Christensen medium and Simons medium)
- 10): Growth on NAC agar medium: negative (nonviable)
- 11): Production of indole: negative
- 12): Voges-Proskauer reaction (Voges-Proskauer test):
negative
- 13): Methyl Red test: negative
- 14): Arginine dihydrolase test: weakly positive
- 15): Lysine decarboxylase test: positive
- 16): Ornithine decarboxylase test: positive
- 17): Esculin hydrolysis: negative
- 18): DNase test: negative
- 19): Starch hydrolysis: negative
- 20): ONPG test (cultivated at 37°C): negative
- 21): Acylamidase test: positive
- 22): Phosphatase test: positive
- 23): Chitin hydrolysis: negative
- 24): Productivity of levan from sucrose: positive
- 25): Productivity of acids and gas from sugars:
Neither acids nor gases were produced from the following 13 sugars: glucose, fructose, galactose, mannose, xylose, arabinose, maltose, lactose, rhamnose, sucrose, cellobiose, trehalose and mannitol.
- 26): Accumulation of poly-β-hydroxybutyrate in the cell: negative
- 27): Utilization of carbon sources:
On the medium containing minerals, glucose and calcium 2-keto-gluconate can be used as a sole carbon source for the formation of the cells. In this case, it seemed that specific vitamins for growth were not required. On the other hand, D-(+)-trehalose, DL-arginine, geraniol, β-alanine, L-valine and inositol were not be utilized.
- 28): G+C mole % (HPLC method): 60.4% (A+T mole % = 39.6%)

In view of the above test results, PBJ-5360-STR-1-21 is an aerobic Gram-negative rod and moves actively in a liquid medium using one or more polar flagella. It is positive for catalase and contains oxidase. It was negative for OF-test (showing to be alkaline). In view of these observations, it is apparent that the present bacterium belongs to the genus *Pseudomonas* in Family *Pseudomonadaceae*.

When the inventors compared the above properties with those of the bacterial complexes which are incapable of accumulating poly-β-hydroxybutyrate (PHB) in their cells, which complexes are described in Bergey's Manual of Systematic Bacteriology, Vol. 1 (1984) on Genus *Pseudomonas*, the present inventors failed to find any bacterium having those properties consistent or analogous to the properties described above. This bacterium appears a considerably unusual strain of *Pseudomonas* because it hydrolyzes arginine and decarboxylates lysine and ornithine. The G+C mole % value of 60.4% indicates that the strain belongs to a group having lower G+C value in *Pseudomonas*. Thus, in view of the various properties mentioned above, the present bacterium has been identified as *Pseudomonas* sp. PBJ-5360-STR-1-21. These properties were consistent with those of the parent strain PBJ-5360.

## Claims

1. Antibiotic stalobacin selected from the group consisting of stalobacins H and I having physico-chemical properties as shown below:
| | Stalobacin H | Stalobacin I |
|---|---|---|
| m.p. (°C) (as Na salt) | 235°C (dec.) | 240°C (dec.) |
| LSI-MS Protonated | 1396 | 1325 |
| molecular ion (m/z) | | |
| IR (KBr) (cm⁻¹) | 3374, 1747, | 3387, 1747, |
| | 1654, 1597, 1525 | 1651, 1596, 1527 |
| UV (H₂O) | End absorption | End absorption |
| CD (H₂O) | [θ]₁₉₄-66980 | |
| | [θ]₂₁₂+9851 | [θ]₂₀₆+11530 |
| | [θ]₂₃₂-31520 | [θ]₂₃₂-28660 |
| | [θ]₂₅₇-4288 | [θ]₂₅₇+4749 |
| Retention time (min.) | 8.8 | 9.7 |
| in HPLC* | | |
| Amino Acid Analysis (molar ratio) | | |
| HyAsp¹⁾ | HyAsp (1) | HyAsp (1) |
| Asp | Asp (1) | Asp (1) |
| Ser | Ser (1) | Ser (1) |
| HyIle²⁾ | HyIle (1) | HyIle (1) |
| Gly | Gly (1) | Gly (1) |
| Ala | Ala (1) | --- |
| | | |
|---|---|---|
| * Column: Develosil 5C18, 4.6 i.d. x 250 mm Mobile phase: CH₃CN/2 mM H₃PO₄ (containing 50 mM Na₂SO₄) = 43/57 Flow rate: 1 ml/min. | | |
| 1) Hydroxyaspartic acid | | |
| 2) Hydroxyisoleucine | | |

2. *Pseudomonas* sp. PBJ-5360-STR-1-21 which was deposited under accession No. FERM P-14149 with the National Institute of Bioscience and Human Technology, Higashi 1-1-3, Tsukubashi, Ibaraki pref. Japan, on February 16, 1994, said original deposit being transferred to International deposition under the Budapest Treaty on April 28, 1994, and given Accession number FERN BP-4661, and which produces antibiotic stalobacins as described in claim 1.

3. Process for producing antibiotic stalobacins defined in claim 1 which comprises cultivating *Pseudomonas* sp. PBJ-5360-STR-1-21 which was deposited under accession No. FERM P-14149 with the National Institute of Bioscience and Human Technology, Higashi 1-1-3, Tsukubashi, Ibaraki pref. Japan, on February 16, 1994, said original deposit being transferred to International deposition under the Budapest Treaty on April 28, 1994, and given Accession number FERM BP-4661 to produce antibiotic stalobacins as described in claim 1 and separating and recovering the antibiotic stalobacins from the culture.

## Patentansprüche

1. Antibiotisches Stalobacin ausgewählt aus der Gruppe bestehend aus Stalobacin H und Stalobacin I mit den folgenden physikalisch-chemischen Eigenschaften:
| | Stalobacin H | Stalobacin I |
|---|---|---|
| Smp. (°C) (als Natriumsalz | 235°C (Zers.) | 240°C (Zers.) |
| LSI-MS protoniertes | 1396 | 1325 |
| Molekülion (m/z) | | |
| IR (KBr) (cm⁻¹) | 3374, 1747, | 3387, 1747, |
| | 1654, 1597, 1525 | 1651, 1596,1527 |
| UV (H₂O) | Endabsorption | Endabsorption |
| CD (H₂O) | [θ]₁₉₄- 66980 | |
| | [θ]₂₁₂+9851 | [θ]₂₀₆+11530 |
| | [θ]₂₃₂-31520 | [θ]₂₃₂-28660 |
| | [θ]₂₅₇-4288 | [θ]₂₅₇+4749 |
| Retentionszeit (min) in HPLC* | 8,8 | 9,7 |
| Analyse der Aminosäuren | | |
| (molares Verhältnis) | | |
| HyAsp¹⁾ | HyAsp (1) | HyAsp (1) |
| Asp | Asp (1) | Asp (1) |
| Ser | Ser (1) | Ser (1) |
| Hylle²⁾ | Hylle (1) | Hylle (1) |
| Gly | Gly (1) | Gly (1) |
| Ala | Ala (1) | --- |
| | | |
|---|---|---|
| * Säule: Develosil 5C18, 4,6 i.D. x 250 mm Mobile Phase: CH₃CN/2 mM H₃PO₄ (enthaltend 50 mM Na₂SO₄) = 43/57 Fließrate: 1 ml/min | | |
| 1) Hydroxyasparaginsäure | | |
| 2) Hydroxyisoleucin | | |

2. *Pseudomonas* sp. PBJ-5360-STR-1-21, das beim National Institute of Bioscience and Human Technology, Higashi 1-1-3, Tsukubashi, Ibaraki pref. Japan am 16. Februar 1994 mit der Hinterlegungsnummer FERM P-14149 hinterlegt wurde, wobei die ursprüngliche Hinterlegung am 28. April 1994 in eine internationale Hinterlegung gemäß dem Budapester Vertrag unter der Hinterlegungsnummer FERM BP-4661 umgewandelt wurde, und das antibiotische Stalobacine nach Anspruch 1 produziert.

3. Verfahren zur Herstellung von antibiotischen Stalobacinen nach Anspruch 1, umfassend das Züchten von *Pseudomonas* sp. PBJ-5360-STR-1-21, das beim National Institute of Bioscience and Human Technology, Higashi 1-1-3, Tsukubashi, Ibaraki pref. Japan am 16. Februar 1994 mit der Hinterlegungsnummer FERM P-14149 hinterlegt wurde, wobei die ursprüngliche Hinterlegung am 28. April 1994 in eine internationale Hinterlegung gemäß dem Budapester Vertrag unter der Hinterlegungsnummer FERM BP-4661 umgewandelt wurde, für die Herstellung von antibiotischen Stalobacinen nach Anspruch 1, sowie Trennen und Gewinnen der antibiotischen Stalobacine aus der Kultur.

## Revendications

1. Stalobacine à caractère antibiotique extraite du groupe comprenant des stalobacines H et I ayant les propriétés physico-chimiques ci-dessous :
| | Stalobacine H | Stalobacine I |
|---|---|---|
| p.f. (°C) (comme le sel de sodium) | 235 °C (dec.) | 240 °C (dec.) |
| Ion moléculaire protoné par LSI-MS | 1396 | 1325 |
| (m/z) | | |
| IR (KBr) (cm⁻¹) | 3374, 1747, 1654, | 3387, 1747, 1651, |
| | 1597, 1525 | 1596, 1527 |
| UV (H₂O) | Absorption finale | Absorption finale |
| CD (H₂O) | [θ]₁₉₄-66980 | |
| | [θ]₂₁₂+9851 | [θ]₂₀₆+11530 |
| | [θ]₂₃₂-31520 | [θ]₂₃₂-28660 |
| | [θ]₂₅₇-4288 | [θ]₂₅₇+4749 |
| Temps de rétention (min) en HPLC* | 8,8 | 9,7 |
| Analyse d'acides aminés (rapport | | |
| molaire) | | |
| HyAsp¹⁾ | HyAsp (1) | HyAsp (1) |
| Aap | Asp (1) | Asp (1) |
| Ser | Ser (1) | Ser (1) |
| Hylle²⁾ | Hylle (1) | Hylle (1) |
| Gly | Gly (1) | Gly (1) |
| Ala | Ala (1) | --- |
| | | |
|---|---|---|
| * Colonne : Develosil 5C18, diam.int. 4,6 x 250 mm Phase mobile : CH₃CN/2 mM H₃PO₄ (contenant 50 mM de Na₂SO₄) = 43/57 Débit : 1 ml/min | | |
| 1) Acide hydroxy-aspartique | | |
| 2) Hydroxy-isoleucine | | |

2. *Pseudomonas* sp. PBJ-5360-STR-1-21 qui a été déposée sous le numéro d'accès FERM P-14149 auprès du National Institute of Bioscience and Human Technology, Higashi 1-1-3, Tsukubashi, Ibaraki pref. Japon, le 16 février 1994, ledit dépôt original ayant été transféré au dépôt international conformément au Traité de Budapest le 28 avril 1994, et ayant été doté du numéro d'accès FERM BP-4661, et qui produit des stalobacines à caractère antibiotique décrites dans la revendication 1.

3. Procédé pour produire les stalobacines à caractère antibiotique définies dans la revendication 1, qui comprend la culture de *Pseudomonas* sp. PBJ-5360-STR-1-21 qui a été déposée sous le numéro d'accès FERM P-14149 auprès du National Institute of Bioscience and Human Technology, Higashi 1-1-3, Tsukubashi, Ibaraki pref. Japon, le 16 février 1994, ledit dépôt original ayant été transféré au dépôt international conformément au Traité de Budapest le 28 avril 1994, et ayant été doté du numéro d'accès FERM BP-4661 pour produire les stalobacines à caractère antibiotique décrites dans la revendication 1, la séparation et la récupération des stalobacines à caractère antibiotique de la culture.
